(19) Europäisches Patentamt
European Patent Office
Office européen des brevets

(11) Publication number: **0 098 134**
**B1**

# (12) EUROPEAN PATENT SPECIFICATION

(45) Date of publication of patent specification: **03.06.87**

(51) Int. Cl.$^4$: **C 07 F 15/00,** A 61 K 31/28

(21) Application number: **83303686.6**

(22) Date of filing: **27.06.83**

## (54) Ionic bis(dicarboxylato)palladate(II) anti-tumor agents.

(30) Priority: **28.06.82 US 392817**

(43) Date of publication of application:
**11.01.84 Bulletin 84/02**

(45) Publication of the grant of the patent:
**03.06.87 Bulletin 87/23**

(84) Designated Contracting States:
**AT BE CH DE FR GB IT LI LU NL SE**

(56) References cited:
**GB-A-1 030 046**
**US-A-3 883 570**

(73) Proprietor: **ENGELHARD CORPORATION**
**70 Wood Avenue South CN 770**
**Iselin New Jersey 08830 (US)**

(72) Inventor: **Amundsen, Alan R.**
**12 Meadowbrook Drive**
**Somerville New Jersey (US)**
Inventor: **Stern, Eric W.**
**234 Oak Tree Road**
**Mountainside New Jersey (US)**

(74) Representative: **Colgan, Stephen James et al**
**CARPMAELS & RANSFORD**
**43 Bloomsbury Square**
**London WC1A 2RA. (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 098 134 B1**

Courier Press, Leamington Spa, England.

**Description**

This invention relates to a class of ionic bis(dicarboxylato)palladate(II) compounds and to pharmaceutical compositions comprising them.

There are very few palladium complexes which are useful in the treatment of tumors and it is an object of this invention to add to the known family of anti-tumor agents by providing a new class of ionic palladium compounds.

BACKGROUND

The discovery that platinum complexes are active against tumors has brought about a renewed interest in metal complexes as a source for anti-cancer agents. Cisplatin, cis-[$Pt(NH_3)_2Cl_2$], for example, has been singularly successful in bringing about a regression of testicular and ovarian tumors and, as a result, other platinum derivatives have been investigated for anti-tumor activity.

These development have led to the exploitation of structure-activity relationships including the synthesis of palladium analogs. Most of these efforts have been directed to the preparation of neutral palladium compounds. However, many of these have failed to show any discernible activity and, as a rule, have low solubility in aqueous solutions. Moreover, structure-activity relationships which have been developed for platinum compounds would predict that only neutral compounds would have anti-tumor activity. (M. J. Cleare and J. D. Hoeschele; "Bioinorganic Chemistry", Vol. *2*: page 187 (1973); and M. J. Cleare; "Coordination Chemistry Reviews", Vol. *12*: page 349 (1974)).

THE INVENTION

This invention relates to the use of a class of ionic palladium compounds which are soluble in aqueous solution and which are useful anti-tumor agents in mammals. These compounds exhibit excellent activity against malignant tumor cells in animals as well as low mammalian toxicity.

More specifically, this invention relates to the use of alkali metals and alkaline earth metal bis(dicarboxylato)palladate(II) compounds derived from aliphatic and aromatic dicarboxylic acids. The palladium in said compounds is shared between two rings which contain 6 or 7 atoms per ring.

The compounds of this invention are used for the treatment of malignant tumours and possess the following structural formula:

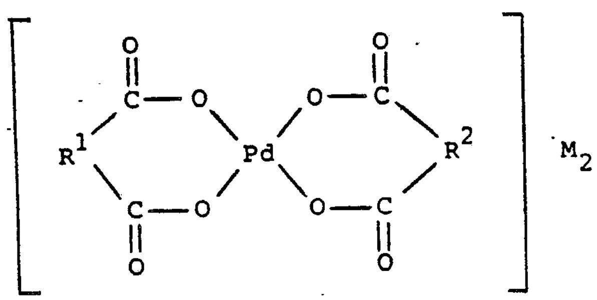

(I)

wherein $R^1$ and $R^2$ are the same or different and represent the following:

methylene or ethylene

cycloalkylene of the formula:

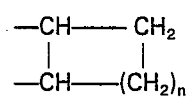

wherein n is an integer having a value of 2—3; alkylidene of 2—4 carbon atoms; cycloalkylidene containing from 4—6 nuclear carbon atoms;

o-phenylene of the formula:

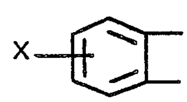

aralkylidene of the formula:

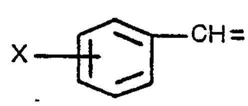

wherein X is selected from hydrogen, halogen and $C_{1-6}$ alkyl;

substituted methylidene of the formula: $=CR^3R^4$

wherein $R^3$ and $R^4$ are selected from hydrogen, halogen and hydroxy with the proviso that $R^3$ and $R^4$ are not simultaneously hydrogen or hydroxy and that when $R^3$ or $R^4$ is halogen then $R^4$ or $R^3$ is not hydroxy; or keto;

M is an alkali metal or $M_2$ is an alkaline earth metal.

Compounds wherein $R^1$ and $R^2$ are both methylene are known from Gmelin's Hambuch Der Anorganischem Chemie, Vol. *65:* page 320 (1847); as well as J.A.C.S., Vol. *81*: page 287 (1959); J. Organic Chemistry, Vol *41*: page 2049 (1976).

The following compounds are a preferred subgroup of pharmacologically active products within the scope of this invention:

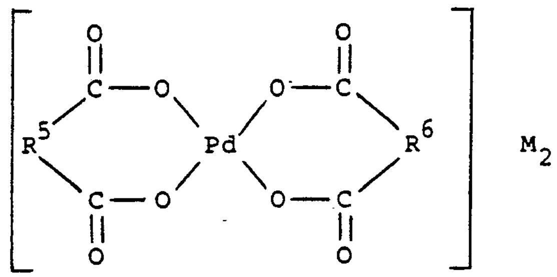

(Ia)

wherein $R^5$ and $R^6$ are identical moieties selected from among:

alkylidene of 2—4 carbon atoms;

cycloalkylidene containing from 4—6 nuclear carbon atoms;

o-phenylene of the formula:

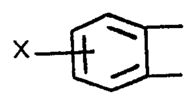

wherein X is selected from hydrogen, halogen and $C_{1-6}$ alkyl;

hydroxymethylidene; and

keto; and

M is an alkali metal.

The aforedescribed products (Ia) exhibit good solubility in aqueous solutions and the dosage levels required for anti-tumor activity is not narrowly critical.

PREPARATIVE METHODS

The products useful in this invention may be prepared by treating an alkali metal or alkaline earth metal carboxylate with a palladium salt. Suitable salts include the palladium halides such as palladium chloride or a functionally equivalent salt having leaving groups similar to halide as, for example, palladium nitrate or a palladium carboxylate such as palladium acetate. Tetrahalopalladates or equivalent salts containing other leaving groups are also suitable. Said palladium salt is added to a concentrated solution of the alkali metal or alkaline earth metal form of the dicarboxylic starting material ($R^1(COOM)_2$,infra) and the mixture is warmed gently or maintained at room temperature over an extended period with stirring. The resulting precipitate is then filtered and washed with a suitable solvent such as ethanol to afford the desired product (I). These products may contain acid residues which are the same or different. The following equation illustrates the preparation of products having identical acid functions or residues but products having a mixed acid functionality may also be obtained by treating said palladium salt with two distinct acids either in the form of a mixture or individually in steps:

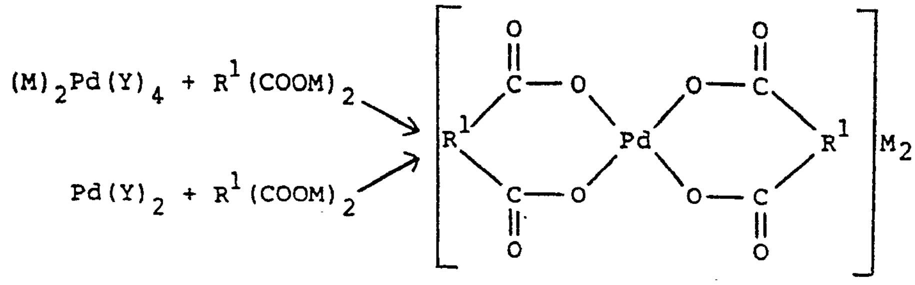

wherein M is an alkali metal or $M_2$ is an alkaline earth metal; Y is a leaving group such as halo, for example, chloro, bromo, nitrato ($NO_3^-$) or alkylcarboxy such as acetoxy; and the two $R^1$ radicals are identical moieties as defined hereinabove.

The dicarboxylate starting materials in the foregoing process are obtained via neutralization of the corresponding dicarboxylic acid precursor with alkali metal hydroxide or an alkaline earth metal hydroxide. If desired, the resulting solution may be evaporated to dryness and the residue recrystallized from water to afford an essentially pure alkali metal or alkaline earth metal dicarboxylate which may be used directly in the afore-described process.

The products of this invention may also be obtained via the reaction of palladium hydroxide with a dicarboxylic acid salt [$R^1(COOM)_2$]. This procedure is illustrated by the following equation in which $R^1(COOM)_2$ is treated with $Pd(OH)_2$ to afford a bis(dicarboxylato)palladate(II) having identical acid (ie., $R^1$) residues:

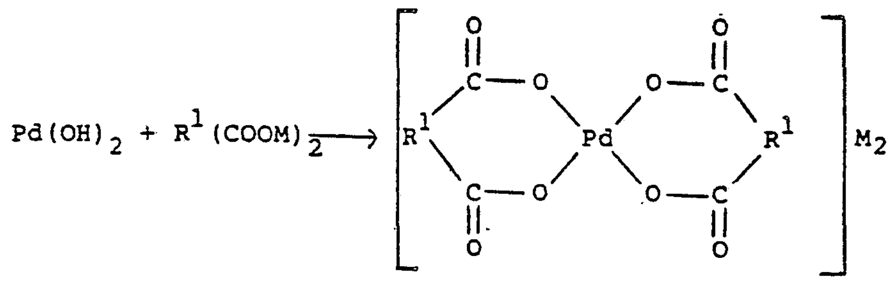

wherein M is as defined above and $R^1$ represents the residue of a dicarboxylic acid as hereinbefore defined. In this instance the acid residues are derived from a single dicarboxylic acid and they are identical. Those products in which the acid functions are dissimilar can be prepared in an essentially identical manner by treating the $Pd(OH)_2$ first with one form of dicarboxylate salt ($R^1COOM$) at half the stoichiometric concentration and then with another ($R^2COOM$) in an identical amount. Temperature is not critical to the afore-described process and ambient temperatures may be employed; however, it is desirable in some instances to facilitate the process by the application of heat and the use of agitation means. The products are obtained from solution in the form of precipitates which may be isolated by filtration and then washed and dried. Recrystallization from alcohol affords an essentially pure product.

## PHARMACOLOGY

These products are useful in the treatment of tumors in animals, as, for example, Sarcoma 180 ascites in mammals such as mice. This anti-tumor effect also may extend to other sarcomas and to such other tumors as the following: lymphoid leukemia, lymphosarcoma, myelocytic leukemia, malignant lymphoma, squamous cell carcinoma, adenocarcinoma, scirrhous carcinoma, malignant melanoma, seminoma, teratoma, choriocarcinoma, embryonalcarcinoma, cystadenocarcinoma, endometroidcarcinoma or neuroblastoma. In addition, said complexes may be useful as anti-viral, anti-inflammatory, anti-bacterial and anti-parasitic agents.

They may be administered parenterally or orally in admixture with a non-toxic pharmacologically acceptable inert carrier or diluent in any of the usual pharmaceutical forms. These include solid and liquid oral unit dosage forms such as tablets, capsules, powders and suspensions or solutions and suspensions for subcutaneous, intramuscular, intravenous or intra-arterial injection.

The term "unit dosage" refers to physically discrete units which may be administered in single or multiple dosages each containing a predetermined quantity of the active ingredient in association with the required diluent, carrier or vehicle. The quantity of active ingredient is the amount of the complex which is needed to produce the desired therapeutic effect.

A typical unit dosage consists essentially of from about 10—500 mg. of active ingredient; however, the form in which said ingredient is administered and the frequency of administration is usually determinative of the concentration. Thus, for example, oral unit dosage forms containing 10—500 mg of active ingredient may be administered one or more times per day depending upon the severity of the tumor which is sought to be treated and the condition of the host animal. By contrast, parenteral administration generally requires from about 20—250 mg. of the active ingredient per unit dosage administered as a daily dose or as a fraction thereof depending upon whether the regimen calls for administration once, twice, three or four times daily.

By contrast to the "unit dosage", the effective dose is that dosage which is needed to achieve the desired anti-tumor effect. In general, this dosage lies within the range of from about 6—1200 mg of the active ingredient per kg of body weight of the host animal. A preferred concentration lies within the range of from about 10—500 mg/kg of body weight. For oral administration is has been found that an effective dose of 30—1200 mg/kg is most suitable, whereas, in the case of parenteral administration it is usually advisable to employ from about 6—200 mg/kg. These dosages are well below the toxic or lethal dose and they may be varied over a wide range for adjustment to the patient which is being treated.

In this invention the term "pharmacologically acceptable inert carrier or diluent" denotes a non-toxic substance which, when mixed with the active ingredient, renders it more suitable for administration. Compositions intended for oral administration may include such carriers or diluents as corn starch, potato

starch, sodium carboxymethyl cellulose, ethyl cellulose, cellulose acetate, powdered gum tragacanth, gelatin, alginic acid, agar, stearic acid or the sodium, calcium and magnesium salts of stearic acid, sodium lauryl sulfate, polyvinylpyrrolidone, sodium citrate, calcium carbonate and dicalcium phosphate. Said compositions may also contain non-toxic adjuvants and modifiers such as dyes, buffering agents, preservatives, surfactants, emulsifiers, flavoring agents or biocides.

Tablets are prepared by mixing a complex of this invention in a suitably comminuted or powdered form with a diluent or base such as starch, kaolin or di-calcium phosphate. The resulting mixture can be granulated by wetting with a binder such as a syrup, starch (paste), acacia mucilage or solutions of cellulosic or polymeric materials, whereafter, the wetted mixture is forced through a screen. As an alternative to granulating, the powdered mixture can be run through a tablet machine and imperfectly formed slugs broken into granules. The granules are lubricated to prevent sticking to the tablet-forming dies via the addition of stearic acid, a stearate salt, talc or mineral oil and the lubricated mixture is then compressed into tablets. The complexes can also be combined with free flowing inert carriers followed by compression into tablets without going through the granulating or slugging steps. A protective coating or sealing coat of shellac, sugar or polymeric material and a polished coating of wax can also be provided. Dyestuffs may be added to distinguish different unit dosages.

Capsules are formulated by preparing a powdered mixture, according to the procedure hereinbefore described and pouring said mixture into preformed gelatin sheaths. A lubricant such as talc, magnesium stearate or calcium stearate can be added as an adjuvant prior to the filling operation. A glidant such as colloidal silica may be added to improve the flow characteristics and a disintegrating or solubilizing agent may also be added to enhance the effectiveness of the medicament upon ingestion.

Powders are prepared by comminuting the compound to a fine size and mixing with a similarly comminuted pharmaceutical diluent or carrier such as an edible carbohydrate as, for example, starch. Sweetening agents and flavorings, preservatives and dispersing and/or coloring agents may also be employed.

Oral fluids such as syrups and elixirs are prepared in unit dosage form so that a given quantity of medicament, such as a teaspoonful, will contain a predetermined amount of the active ingredient. Suspensions can be formulated by dispersing the active ingredient in a non-toxic vehicle in which it is essentially insoluble.

Fluid unit dosage forms for parenteral administration can be prepared by placing a measured amount of the complex in an ampoule or vial which is sterilized and sealed. An accompanying vial or vehicle can be provided for mixing with said complex prior to administration.

This invention also provides for combining two or more of the subject complexes into a single unit dosage form or, alternatively, combining one or more of said complexes with other known anti-tumor agents, therapeutic agents or nutritive agents so as to enhance or complement the anti-tumor effect.

The preferred compositions for oral administration are tablets in which the lactate complex is present in quantities of 10—500 mg. but, preferably, 50—300 mg. in a pharmaceutically acceptable orally ingestible solid carrier. If desired, the composition may also contain flavors, binders, lubricants and other excipients known in the art.

A preferred alternative for oral administration is the soft gelatin capsule. Such a composition may contain from 10—500 mg. but, preferably, 50—300 mg. by weight of active ingredient dissolved or suspended in vegetable oil, peanut oil, alcohol or glycerine.

The following embodiments illustrate representative unit dosage forms:

Compressed Tablet

A tablet suitable for swallowing is prepared by mixing the following ingredients:

| | |
|---|---|
| Potassium Bis(ethylmalonato)palladate(II) | 150 mg. |
| Niacinamide | 50 mg. |
| Calcium Pantothenate | 20 mg. |
| Magnesium Sulfate | 50 mg. |
| Zinc Sulfate | 50 mg. |
| Magnesium Stearate | 10 mg. |
| | 330 mg. |

The potassium bis(ethylmalonato)palladate(II), niacinamide, calcium pantothenate, magnesium sulfate, zinc sulfate and magnesium stearate (5.0 mg.) are mixed and compressed into slugs. The slugs are then broken into granules and sifted through a screen. Additional magnesium stearate (5.0 mg.) is added and the mixture is then compressed into tablets suitable for oral administration.

Soft Gelatin Capsule

A soft elastic gelatin capsule is filled with the following ingredients:

| | |
|---|---|
| Potassium Bis(Phthalato)palladate(II) | 200 mg. |
| Wheat germ oil | 50 mg. |
| Sunflower seed oil | 100 mg. |
| | 350 mg. |

The potassium bis(phthalato)palladate(II) and wheat germ oil are mixed with sunflower seed oil and the resulting mixture is poured into gelatin capsules suitable for oral administration. An alternative embodiment provides for substituting sunflower seed oil and wheat germ oil with equal amounts of peanut oil to obtain an otherwise similar capsule which is also suitable for oral administration.

Dry Filled Capsule

A hard dry-filled capsule may be prepared from the following ingredients:

| | |
|---|---|
| Potassium Bis(Ketomalonato)palladate(II) | 150 mg. |
| Niacinamide | 50 mg. |
| Calcium Pantothenate | 10 mg. |
| | 210 mg. |

The potassium bis(ketomalonato)palladate(II) is reduced to a powder. Niacinamide and calcium pantothenate are passed through a sieve and these ingredients are added to the potassium bis(ketomalonato)palladate(II). This combination of ingredients is mixed for 10 minutes and then poured into a gelatin capsule.

Dry Powder

The following composition illustrates a representative dosage in dry powder form. In this embodiment the active ingredient is water soluble and it is combined with up to 60% by weight of a suitable flavoring agent. All quantities are in a weight-percent relationship.

| | |
|---|---|
| Potassium Bis(Phthalato)palladate(II) | 25—90% |
| Flavoring Agent | 10—60% |
| Preservative | 0—1.0% |

The potassium bis(phthalato)palladate(II) flavoring agent and preservative are thoroughly blended to afford a homogeneous dry powder which is readily soluble in water. The resulting formulation may be blended with other therapeutic agents to afford combination-type medicinals. Alternatively, said powder may be dissolved in a pharmacologically acceptable diluent to afford a solution which is suitable for oral administration.

Compositions intended for parenteral administration may include such diluents and carriers as water-miscible solvents as, for example, sesame oil, groundnut oil, and aqueous propylene glycol. Typical of said compositions are solutions which contain the active ingredient in sterile form. An embodiment illustrating a dosage form suitable for intravenous injection is set forth below.

Parenteral Solution

Injectable solutions can be formulated by mixing an ampoule of active ingredient with an ampoule of sterile diluent:

| | |
|---|---|
| Ampoule: Potassium Bis(Cyclobutanedicarboxylato)-palladate(II) | 150 mg |
| Ampoule: Sterile Water (Diluent for Injection) | 2 ml |

The potassium bis(cyclobutanedicarboxylato)palladate(II) and water are mixed thoroughly immediately prior to administration. If desired, one or more other active ingredients may be added to

provide an injectable solution having enhanced therapeutic activity.

The following embodiments illustrate the methods by which the products (I) of this invention are obtained; however, it is to be understood that said embodiments are merely illustrative and they are not to be construed as being limitative of the invention herein described and claimed.

PREFERRED EMBODIMENTS

Example 1

Potassium Bis(Ethylmalonato)palladate(II)

Ethylmalonic acid (1.98g, 15 mmoles) and potassium hydroxide (1.68g, 30 mmoles) were dissolved in water (15 ml) and solid palladium chloride (1.065g, 6 mmoles) was added. The mixture was stirred for 30 minutes and the resulting solution was added to ethanol (100 ml) and placed in a freezer for 2.5 hours. The product was filtered, washed with two 15 ml portions of ethanol and vacuum dried to afford 1.48g (55.5%) yield of potassium bis(ethylmalonato)palladate(II). This product was soluble in water in an amount of 70 mg/ml at room temperature. Palladium analysis for $K_2PdC_{10}H_{12}O_8,H_2O$:

Calculated: 22.99%
Found: 22.24%

Example 2

Potassium Bis(Ketomalonato)palladate(II)

Ketomalonic acid (0.68g, 5mmoles) was dissolved in 1M potassium hydroxide (10 ml, 10 mmoles) and palladium chloride (0.355g, 2mmoles) was added. The mixture was stirred for 1.5 hours and the resulting solution was cooled to 0°C. A yellow precipitate formed. An equal volume of ethanol was added and the precipitate was filtered, washed with three 10 ml portions of ethanol and vaccum dried. There was thus obtained 0.57g (68.1% of potassium bis(ketomalonato)palladate(II). This product was soluble in water in an amount of 6.7 mg/ml at room temperature. Palladium analysis for $K_2PdC_6O_{10}.5H_2O$:

Calculated: 20.99%
Found: 20.71%

Example 3

Potassium Bis(Methylmalonato)palladate(II)

Methylmalonic acid (0.59g, 5 mmoles) was dissolved in 1M potassium hydroxide (10 ml, 10 mmoles) and palladium chloride (0.355g, 2mmoles) was added. The mixture was stirred at room temperature for 45 minutes during which time the palladium chloride dissolved and a yellow precipitate formed. This precipitate was filtered washed with 10 ml of water and two 10 ml portions of ethanol and then vacuum dried. The yield of potassium bis(methylmalonato)palladate(II) was 0.42g (50.4%). This product was soluble in water in an amount of 21 mg/ml at room temperature. Palladium analysis for $K_2PdC_8H_8O_8 \cdot 2H_2O$:

Calculated: 23.50%
Found: 23.75%

Example 4

Potassium Bis(Tartronato)palladate(II)

Tartronic acid (0.60g, 5 mmoles) was dissolved in 1M potassium hydroxide (10 ml, 10 mmoles) and solid palladium chloride (0.355g, 2 mmoles) was added. The mixture was stirred at room temperature for one hour during which time a yellow precipitate formed. This precipitate was filtered, washed with water (10 ml) and two 10 ml portions of ethanol and vacuum dried. The yield of potassium bis(tartronato)palladate(II) was 0.75g (98.3%). This product was soluble in water in an amount of 2 mg/ml at room temperature. Palladium analysis for $K_2PdC_6H_4O_{10}.2H_2O$:

Calculated: 25.05%
Found: 24.67%

Example 5

Potassium Bis(Phthalato)palladate(II)

Phthalic acid (0.83g, 5 mmoles) was dissolved in 1 M potassium hydroxide (10 ml, 10 mmoles) and solid palladium chloride (0.355g, 2 mmoles) was added. This mixture was stirred at room temperature for 1.75 hours and it resulted in the formation of a reddish solution. Acetone (100 ml) was added and the solution was placed in a freezer overnight. A yellow precipitate formed and this material was filtered, washed with three 10 ml portions of acetone and vacuum dried. There was thus obtained 0.86g (83.9%) of potassium bis(phthalato)palladate(II). This product was soluble in water to the extent of 80 mg/ml at room temperature. Palladium analysis for $K_2PdC_{16}H_8O_8$:

Calculated: 20.75%
Found: 23.67%

Example 6

Potassium Bis(Cyclobutanedicarboxylato)palladate(II)

1,1-Cyclobutanedicarboxylic acid (1.44g, 10 mmoles) was suspended in water (15 ml) and potassium hydroxide (1.12g, 20 mmoles) was added. Upon dissolution of said acid solid palladium chloride (0.71g, 4

mmoles) was added and the mixture was stirred at room temperature for 2.5 hours. Ethanol (50 ml) was added and the mixture was cooled to 0°C to afford a yellow precipitate. This precipitate was filtered, washed with two 10 ml portions of ethanol and vacuum dried. The yield of potassium bis(cyclobutanedicarboxylato)palladate(II) was 1.34g (71.5%). This product was soluble in water in an amount of 200 mg/ml at room temperature. Palladium analysis for $K_2PdC_{12}H_{12}O_8.3H_2O$:

Calculated: 20.34%
Found: 20.01%.

Example 7
Potassium Bis(Dimethylmalonato)palladate(II)

Dimethylmalonic acid (1.32g, 10 mmoles) was suspended in water (10 ml) and potassium hydroxide (1.12g, 20 mmoles) was added. Palladium chloride (0.71g, 4 mmols) was added to the resulting solution and the mixture was stirred at room temperature for two hours. Ethanol (50 ml) was added and the mixture was refrigerated for 30 minutes. The resulting tan precipitate was filtered washed with two 10 ml portions of ethanol and vacuum dried. The yield of potassium bis(dimethylmalonato)palladate(II) was 1.55 g (87.2%). This product was soluble in water in an amount of 300 mg/ml at room temperature. Palladium analysis for $K_2PdC_{10}H_{12}O_8.4H_2O$:

Calculated: 20.50%
Found: 20.73%.

By repeating the procedure of Example 1, using dicarboxylic acids other than ethylmalonic acid, a variety of bis(dicarboxylato)palladate(II) products are obtained. The following equation and accompanying Table illustrate the method of Example 1, the starting materials which may be employed and the products obtained thereby:

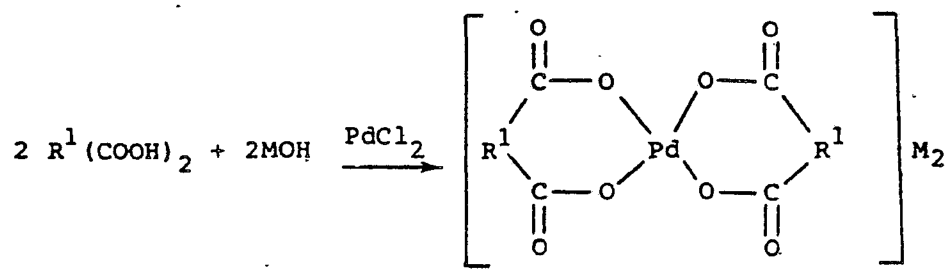

TABLE 1

| Example | $R^1$ | M |
|---|---|---|
| 8 | $—CH(C_2H_5)—$ | Na |
| 9 | $—CH(OH)—$ | Li |
| 10 | $—C(CH_2)_3—$ | Ca |
| 11 | $—C(CH_2)_4—$ | K |
| 12 | $—C(CH_2)_5—$ | K |
| 13 | $—CH(C_6H_5)^*—$ | K |
| 14 | $—CH[—CH(CH_3)_2]—$ | K |
| 15 | $—CHCl—$ | K |
| 16 | $—CCl_2—$ | K |
| 17 | $—CH_2CH_2—$ | K |
| 18 | 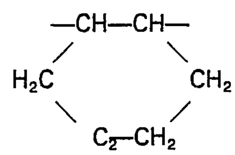 | K |
| 19 | 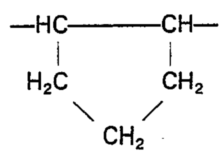 | K |
| 20 | 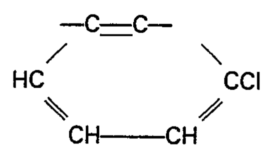 | K |

$^*C_6H_5$ represents phenyl.

Those products in which the acid functions are dissimilar may be obtained by adding $PdCl_2$ to a mixture of potassium dicarboxylates. This method, the starting materials employed and the resulting palladates(II) are illustrated by the following equation and accompanying Table:

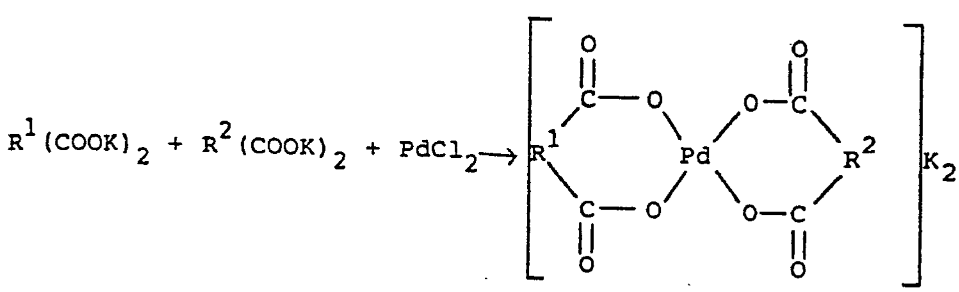

TABLE 2

| Example | $R^1$ | $R^2$ |
|---|---|---|
| 21 | $—CH_2—$ | $—CH(C_2H_5)—$ |
| 22 | $—CH(CH_3)—$ | $—CH(OH)—$ |
| 23 | $—CH(CH_3)_2—$ | $—CH(C_6H_5)$*— |
| 24 | $—CH(CH_2)_5—$ | $—CHCl—$ |

*$C_6H_5$ represents phenyl.

ANTI-TUMOUR EVALUATION

The compounds used in this invention were evaluated against S180 ascites in female CFW Swiss mice. The mice were weighed (average weight: 20 g), placed into cages (six-mice to a cage) and on day zero the mice were inoculated with 0.2 ml of a freshly prepared saline suspension (0.15 M NaCL) containing $1 \times 10^7$ tumor cells/ml or a total of $2 \times 10^6$ cells. This inoculum was freshly prepared using "transfer" mice which had been injected with tumor cells the previous week; it was obtained via the following steps: (1) the removal of cells from the peritoneal cavity of the sacrificed transfer mouse, (2) alternate centrifugation and washing operations (2—3 times with cold saline) to remove blood and other components, and (3) dilution of the volume of the packed cell with saline (1:3). A final centrifugation was carried out at 1000 RPM over a two minute period. A cell count was made on a 2,000-fold dilution of this 1:3 suspension by means of a Coulter Counter. A final dilution to $1 \times 10^7$ cells/ml was made based on the average count.

On day 1, solutions of the test compounds were prepared and each mouse in a set of six was injected with the same test compound at the same dosage. The doses were based on the average weight of the animals (cage weight). Also, beginning on day 1 two controls were employed containing six mice per control:

(1) Normal Control: This consisted solely of the carrier or diluent used in combination with the test compound; and

(2) Positive Control: This consisted solely of the known anti-tumor agent cis-$[Pt(NH_3)_2Cl_2]$ in saline (8 mg/kg) to test the response of the biological system.

The effectiveness of a test compound was measured in terms of the % increase in life span (%ILS) of the test mice relative to the Normal Control (Calculated from the day of tumor inoculation, ie., day zero). To standardize the test data and permit intercomparisons, the day of evaluation was arbitrarily taken as that day corresponding to twice the mean life span (or average day of death) of the control. This established a practical upper limit of 100% on the %ILS attainable. For calculation purposes the survivors on the day of evaluation were considered to have died on that day. The %ILS was calculated as follows:

$$\%ILS = \left( \frac{\text{mean-life span of test mice}}{\text{mean-life span of control mice}} - 1 \right) \times 100$$

ILS values in excess of 50% were interpreted as being indicative of anti-tumor activity, whereas, values in excess of 75% indicated excellent activity.

The test compounds were evaluated in water. The results of these tests are shown in Table 3:

TABLE 3

Anti-Tumor Screening Data; S 180 Ascites

| Example; (Compound) | Dose (mg/kg) | % ILS | Survivors | Positive % ILS | Control Survivors |
|---|---|---|---|---|---|
| Ex. 1 $K_2$[Pd(Ethyl-malonate)$_2$] | 5 | 6 | 0 of 6 | 41 | 0 of 6 |
| | 10 | −3 | 0 of 6 | | |
| | 20 | −7 | 0 of 6 | | |
| | 40 | 9 | 0 of 6 | | |
| | 80 | −6 | 0 of 6 | | |
| | 160 | 94 | 5 of 6 | | |
| | 320 | −13 | 0 of 6 | | |
| | 10 | −3 | 0 of 6 | 52 | 1 of 6 |
| | 20 | 4 | 0 of 6 | | |
| | 40 | −6 | 0 of 6 | | |
| | 80 | 16 | 0 of 6 | | |
| | 160 | 55 | 1 of 6 | | |
| | 320 | −31 | 1 of 6 | | |
| Ex. 2 $K_2$[Pd(Keto-malonate)$_2$] | 10 | 4 | 0 of 6 | 41 | 0 of 6 |
| | 20 | 4 | 0 of 6 | | |
| | 40 | 4 | 0 of 6 | | |
| | 80 | 13 | 1 of 6 | | |
| | 160 | 81 | 5 of 6 | | |
| | 320 | 17 | 0 of 6 | | |
| Ex. 3 $K_2$[Pd(Methyl-malonate)$_2$] | 6.25 | 11 | 0 of 6 | 61 | 2 of 6 |
| | 12.5 | 2 | 0 of 6 | | |
| | 25 | −6 | 0 of 6 | | |
| | 50 | 2 | 0 of 6 | | |
| | 100 | 54 | 2 of 6 | | |
| | 200 | 54 | 0 of 6 | | |
| | 400 | −83 | 0 of 6 | | |
| Ex. 4 $K_2$[Pd(Tartron-ate)$_2$] | 12.5 | 1 | 0 of 6 | 61 | 2 of 6 |
| | 25 | 12 | 0 of 6 | | |
| | 50 | 11 | 0 of 6 | | |
| | 100 | 68 | 3 of 6 | | |
| | 200 | 76 | 0 of 6 | | |
| | 400 | 14 | 3 of 6 | | |
| Ex. 5 $K_2$[Pd(Phthal-ate)$_2$] | 50 | 18 | 0 of 6 | 91 | 4 of 6 |
| | 100 | 38 | 1 of 6 | | |
| | 200 | 79 | 2 of 6 | | |
| | 400 | 25 | 0 of 6 | | |
| Ex. 6 $K_2$[Pd(Cyclo-butanedicarb-oxylate)$_2$] | 10 | −4 | 0 of 6 | 47 | 0 of 6 |
| | 20 | 9 | 0 of 6 | | |
| | 40 | 7 | 0 of 6 | | |
| | 80 | −7 | 0 of 6 | | |
| | 160 | 50 | 0 of 6 | | |
| | 320 | −75 | 0 of 6 | | |

TABLE 3 (CONTINUED)

Anti-Tumor Screening Data; S 180 Ascites

| Example; (Compound) | Dose (mg/kg) | % ILS | Survivors | Positive % ILS | Control Survivors |
|---|---|---|---|---|---|
| Ex. 7 $K_2$[Pd)Dimethyl-malonate)$_2$] | 10 | −15 | 0 of 6 | 47 | 0 of 6 |
| | 20 | 0 | 0 of 6 | | |
| | 40 | −8 | 0 of 6 | | |
| | 80 | −11 | 0 of 6 | | |
| | 160 | 61 | 1 of 6 | | |
| | 320 | 26 | 0 of 6 | | |
| $K_2$[Pd(Malonate)$_2$] | 10 | 6 | 0 of 6 | 86 | 0 of 6 |
| | 20 | −1 | 0 of 6 | | |
| | 40 | 9 | 0 of 6 | | |
| | 80 | 69 | 3 of 6 | | |
| | 160 | 82 | 2 of 6 | | |
| | 320 | −74 | 0 of 6 | | |
| | 10 | 8 | 0 of 6 | 75 | 2 of 6 |
| | 20 | 8 | 0 of 6 | | |
| | 40 | −12 | 0 of 6 | | |
| | 80 | 66 | 2 of 6 | | |
| | 160 | 86 | 3 of 6 | | |
| | 320 | −74 | 0 of 6 | | |
| $K_2$[Pd(Oxalate)$_2$] | 12.5 | 2 | 0 of 6 | 91 | 4 of 6 |
| | 25 | 33 | 0 of 6 | | |
| | 50 | −7 | 0 of 6 | | |
| | 100 | −9 | 1 of 6 | | |
| | 200 | −71 | 0 of 6 | | |

On the basis of Table 3 it can be concluded that the bis(dicarboxylato)palladate(II) compounds are effective anti-tumor agents.

The effective dose ($ED_{90}$), lethal dose ($LD_{50}$) and therapeutic index (TI) were determined via the method of Miller and Taiter (Reported by R. A. Turner, "Screening Methods in Pharmacology", Academic Press, New York, pages 61—62 (1965)).

The results of this test are shown in Table 4. In this study $Ed_{90}$ represents the dose which causes a 50% increase in life span (ILS) in 90% of the test animals (mice) determined graphically. The $LD_{50}$ value represents the lethal dose to 50% of said animals (Therapeutic Index = $LD_{50}/ED_{90}$).

TABLE 4

Therapeutic Indices; S 180 Ascites

| Example (Compound) | Best %ILS (Dosage) | $ED_{90}$ | $LD_{50}$ | Therapeutic Index |
|---|---|---|---|---|
| Ex. 1 $K_2$[Pd(Ethyl-malonate)$_2$] | 94(160mg/kg) | 150 | 320 | 2.1 |
| Ex. 2 $K_2$[Pd(Keto-malonate)$_2$] | 81(160mg/kg) | 200 | 400 | 2.0 |
| Ex. 3 $K_2$[Pd(Methyl-malonate)$_2$] | 54(100mg/kg) | 160 | 180 | 1.1 |
| Ex. 4 $K_2$[Pd(Tartron-ate)$_2$] | 76(200mg/kg) | 190 | 400 | 2.1 |
| Ex. 5 $K_2$[Pd(Phthal-ate)$_2$] | 7(200mg/kg) | 220 | 500 | 2.3 |
| Ex. 6 $K_2$[Pd(Cyclo-butanedicarb-oxylate)$_2$] | 50(160mg/kg) | 210 | 230 | 1.1 |
| Ex. 7 $K_2$[Pd(Dimethyl-malonate)$_2$] | 61(160mg/kg) | 210 | 280 | 1.3 |
| $K_2$[Pd(Malon-ate)$_2$] | 82(160mg/kg)<br>86(160mg/kg) | 120<br>120 | 230<br>230 | 1.9<br>1.9 |

All compounds (I) exhibit favorable therapeutic indices.

**Claims**

1. A palladium(II) compound of the formula:

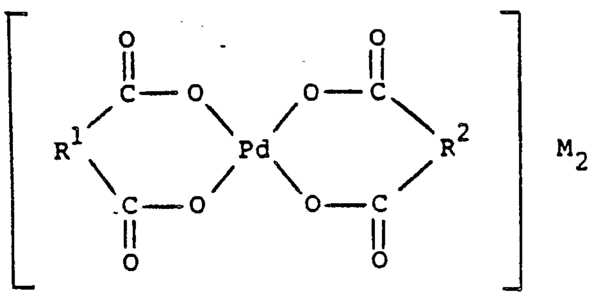

wherein $R^1$ and $R^2$ are the same or different and represent the following:

(a) methylene or ethylene with the proviso that $R^1$ and $R^2$ cannot both represent methylene at the same time.

(b) cycloalkylene of the formula:

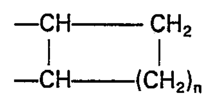

wherein n is an integer having a value of 2—3;

(c) alkylidene of 2—4 carbon atoms;

(d) cycloalkylidene containing from 4—6 nuclear carbon atoms;

(e) o-phenylene of the formula:

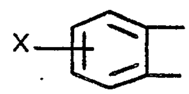

(f) aralkylidene of the formula:

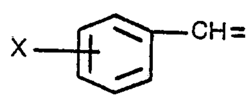

wherein X is selected from hydrogen, halogen and $C_{1-6}$ alkyl;

(g) substituted methylidene of the formula: $=CR^3R^4$

wherein $R^3$ and $R^4$ are selected from hydrogen, halogen and hydroxy with the proviso that $R^3$ and $R^4$ are not simultaneously hydrogen or hydroxy and that when $R^3$ or $R^4$ is halogen then $R^4$ or $R^3$ is not hydroxy; or

(h) keto; and

M is an alkali metal or $M_2$ is an alkaline earth metal.

2. The compound of Claim 1 wherein the palladium(II) compound has a structure of the formula:

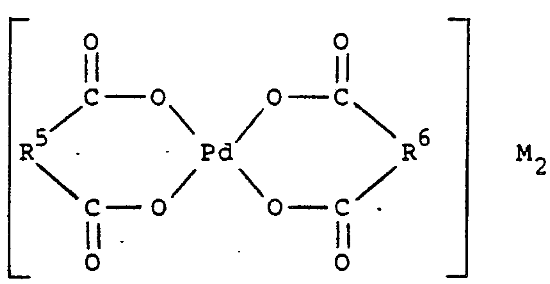

wherein $R^5$ and $R^6$ are identical moieties selected from among:

alkylidene of 2—4 carbon atoms;

cycloalkylidene containing from 4—6 nuclear carbon atoms;

o-phenylene of the formula:

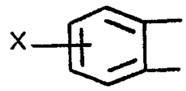

wherein X is selected from hydrogen, halogen and $C_{1-6}$ alkyl;

hydroxymethylidone;

keto; and

M is an alkali metal.

3. The compound of Claim 2 wherein $R^5$ and $R^6$ are selected from ethylidene, o-phenylene, hydroxymethylidene, and keto.

4. A palladium(II) compound for use in the treatment of malignant tumor cells said compound being of the formula:

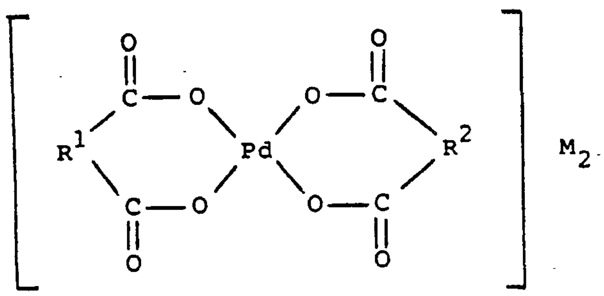

wherein $R^1$ and $R^2$ are the same or different and represent the following:

(a) methylene or ethylene;

(b) cycloalkylene of the formula:

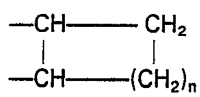

wherein n is an integer having a value of 2—3;

(c) alkylidene of 2—4 carbon atoms;

(d) cycloalkylidene containing from 4—6 nuclear carbon atoms;

(e) o-phenylene of the formula:

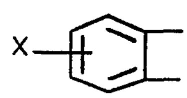

(f) aralkylidene of the formula:

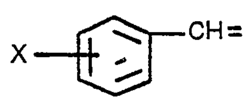

wherein X is selected from hydrogen, halogen and $C_{1-6}$ alkyl;

b (g) substituted methylidene of the formula: $=CR^3R^4$

wherein $R^3$ and $R^4$ are selected from hydrogen, halogen and hydroxy with the proviso that $R^3$ and $R^4$ are not simultaneously hydrogen or hydroxy and that when $R^3$ or $R^4$ is halogen then $R^4$ or $R^3$ is not hydroxy; or

(h) keto; and

M is an alkali metal or $M_2$ is an alkaline earth metal.

5. A compound according to Claim 4 wherein $R^1$ and $R^2$ are identical.

6. A pharmaceutical composition comprising a palladium(II) compound of the formula

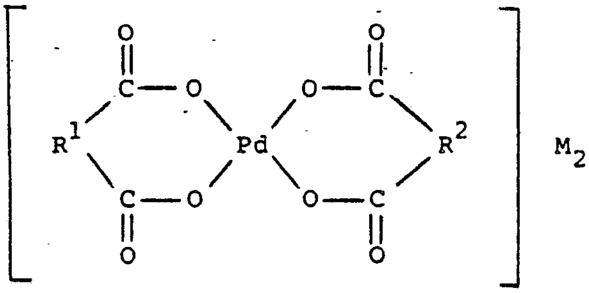

wherein $M_2$, $R^1$ and $R^2$ are as defined in claim 4 or claim 5, and a pharmacologically acceptable inert carrier or diluent.

7. A method of preparing a palladium(II) compound of the formula:

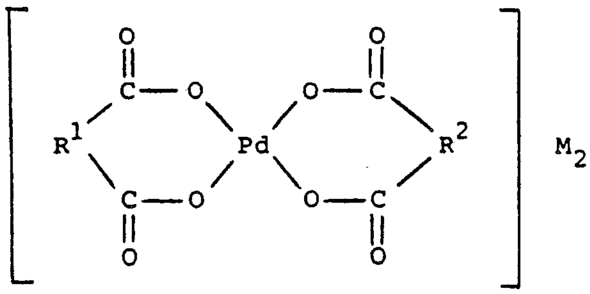

wherein $R^1$ and $R^2$ are the same or different and represent the following:

(a) methylene or ethylene with the proviso that $R^1$ and $R^2$ cannot both represent methylene at the same time.

(b) cycloalkylene of the formula:

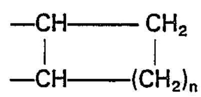

wherein n is an integer having a value of 2—3;

(c) alkylidene of 2—4 carbon atoms;

(d) cycloalkylidene containing from 4—6 nuclear carbon atoms;

(e) o-phenylene of the formula:

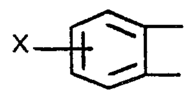

(f) aralkylidene of the formula:

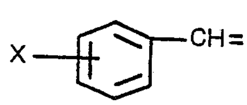

wherein X is selected from hydrogen, halogen and $C_{1-6}$ alkyl;

(g) substituted methylidene of the formula: $=CR^3R^4$

wherein $R^3$ and $R^4$ are selected from hydrogen, halogen and hydroxy with the proviso that $R^3$ and $R^4$ are not simultaneously hydrogen or hydroxy and that when $R^3$ or $R^4$ is halogen then $R^4$ or $R^3$ is not hydroxy; or

(h) keto; and

M is an alkali metal or $M_2$ is an alkaline earth metal;

which method comprises treating dicarboxylate starting material $R^1$ $(COOM)_2$, or $R^1$ $(COOM)_2$ and $R^2$ $(COOM)_2$ where $R^1$ and $R^2$ are different, with a palladium salt.

8. A method of preparing a pharmaceutical composition comprising a palladium(II) compound of the formula given in claim 7, and a pharmacologically acceptable inert carrier or diluent; in which method a

palladium (II) compound of the formula given in claim 7 and a pharmacologically acceptable inert carrier or diluent are mixed with one another.

9. Use of palladium(II) compound of the formula given in claim 7 in the preparation of a medicament for the treatment of tumors.

**Patentansprüche**

1. Eine Palladium(II)-Verbindung der Formel:

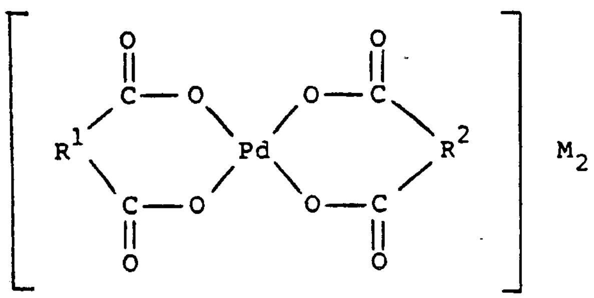

worin $R^1$ und $R^2$ gleich oder verschieden sind und die folgende Bedeutung aufweisen:

(a) Methylen oder Ethylen, mit der Maßgabe, daß $R^1$ und $R^2$ nicht beide gleichzeitig Methylen darstellen können;

(b) Cycloalkylen der Formel:

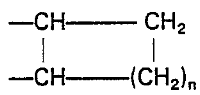

worin n eine ganze Zahl mit einem Wert von 2—3 aufweist;

(c) Alkyliden mit 2—4 Kohlenstoffatomen;

(d) Cycloalkyliden mit 4 bis 6 Ringkohlenstoffatomen;

(e) o-Phenylen der Formel;

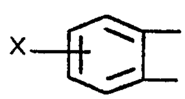

(f) Aralkyliden der Formel

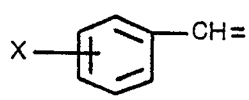

worin X unter Wasserstoff, Halogen und $C_{1-6}$-Alkyl ausgewählt ist;

(g) Substituiertes Methyliden der Formel: $=CR^3R^4$, worin $R_3$ und $R_4$ ausgewählt sind unter Wasserstoff, Halogen und Hydroxy, mit der Maßgabe, daß $R^3$ und $R^4$ nicht gleichzeitig Wasserstoff oder Hydroxy bedeuten und daß dann, wenn $R_3$ oder $R_4$ Halogen darstellt, $R^4$ oder $R^3$ nicht Hydroxy bedeutet; oder

(h) Keto; und

M ein Alkalimetall oder $M_2$ ein Erdalkalimetall bedeutet.

2. Verbindung nach Anspruch 1, worin die Palladium(II)-Verbindung eine Struktur der Formel:

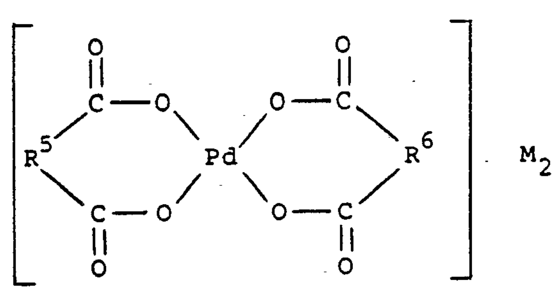

hat,

worin $R^5$ und $R^6$ identische Reste darstellen, ausgewählt unter:
Alkyliden mit 2 bis 4 Kohlenstoffatomen;
Cycloalkyliden mit 4—6 Ringkohlenstoffatomen;
o-Phenylen der Formel:

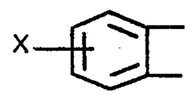

worin X ausgewählt ist unter Wasserstoff, Halogen und $C_{1-6}$-Alkyl;
Hydroxymethyliden;
Keto; und
M ein Alkalimetall bedeutet.

3. Verbindung nach Anspruch 2, worin $R^5$ und $R^6$ ausgewählt sind unter Ethyliden, o-Phenylen, Hydroxymethyliden und Keto.

4. Palladium(II)-Verbindung zur Anwendung in der Bekämpfung maligner Tumorzellen, welche Verbindung der Formel:

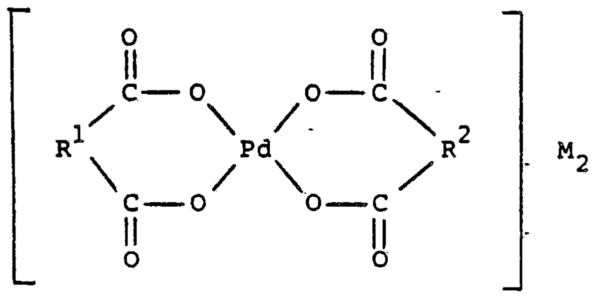

entspricht, worin $R^1$ und $R^2$ gleich oder verschieden sind und die folgende Bedeutung aufweisen:
(a) Methylen oder Ethylen;
(b) Cycloalkylen der Formel:

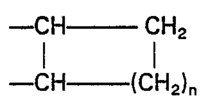

worin n eine ganze Zahl mit einem Wert von 2—3 aufweist;
(c) Alkyliden mit 2—4 Kohlenstoffatomen;
(d) Cycloalkyliden mit 4 bis 6 Ringkohlenstoffatomen;
(e) o-Phenylen der Formel:

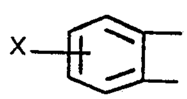

(f) Aralkyliden der Formel:

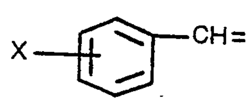

worin X unter Wasserstoff, Halogen und $C_{1-6}$-Alkyl ausgewählt ist;
(g) Substituiertes Methyliden der Formel: $=CR^3R^4$, worin $R_3$ und $R_4$ ausgewählt sind unter Wasserstoff, Halogen und Hydroxy, mit der Maßgabe, daß $R^3$ und $R^4$ nicht gleichzeitig Wasserstoff oder Hydroxy bedeuten und daß dann, wenn $R^3$ oder $R^4$ Halogen darstellt, $R^4$ oder $R^3$ nicht Hydroxy bedeutet; oder
(h) Keto; und
M ein Alkalimetall oder $M_2$ ein Erdalkalimetall bedeutet.

5. Verbindung nach Anspruch 4, worin $R^1$ und $R^2$ identisch sind.

6. Pharmazeutische Zusammensetzung, umfassend eine Palladium(II)-Verbindung der Formel:

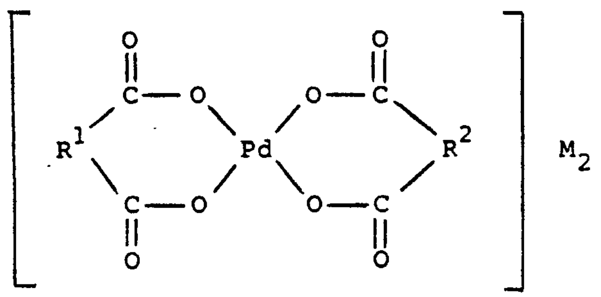

worin $M_2$, $R^1$ und $R^2$ wie in Anspruch 4 oder Anspruch 5 definiert sind, und einen pharmakologisch annehmbaren inerten Träger oder eine solches Verdünnungsmittel.

7. Verfahren zur Herstellung einer Palladium(II)-Verbindung der Formel:

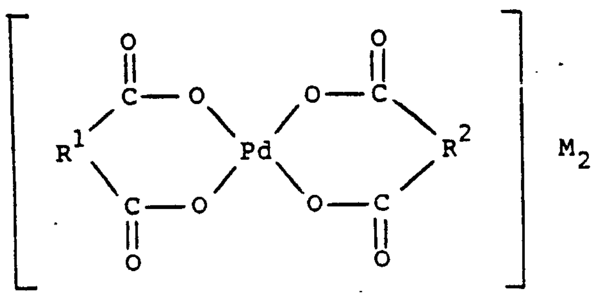

worin $R^1$ und $R^2$ gleich oder verschieden sind und die folgende Bedeutung aufweisen:

(a) Methylen oder Ethylen, mit der Maßgabe, daß $R^1$ und $R^2$ nicht beide gleichzeitig Methylen darstellen können;

(b) Cycloalkylen der Formel:

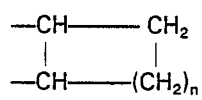

worin n eine ganze Zahl mit einem Wert von 2—3 aufweist;

(c) Alkyliden mit 2—4 Kohlenstoffatomen;

(d) Cycloalkyliden mit 4 bis 6 Ringkohlenstoffatomen;

(e) o-Phenylen der Formel:

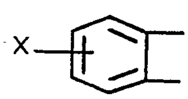

(f) Aralkyliden der Formel:

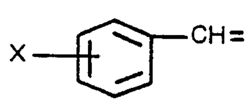

worin X unter Wasserstoff, Halogen und $C_{1-6}$-Alkyl ausgewählt ist;

(g) Substituiertes Methyliden der Formel: $=CR^3R^4$, worin $R_3$ und $R_4$ ausgewählt sind unter Wasserstoff, Halogen und Hydroxy, mit der Maßgabe, daß $R^3$ und $R^4$ nicht gleichzeitig Wasserstoff oder Hydroxy beuten und daß dann, wenn $R_3$ oder $R_4$ Halogen darstellt, $R^4$ oder $R^3$ nicht Hydroxy bedeutet; oder

(h) Keto; und

M ein Alkalimetall oder $M_2$ ein Erdalkalimetall bedeutet;

welches Verfahren ein Behandeln eines Dicarboxylat-Ausgangsmaterials $R^1(COOM)_2$ oder $R^1(COOM)_2$ und $R^2(COOM)_2$, worin $R^1$ und $R^2$ verschieden sind, mit einem Palladium-Salz umfaßt.

8. Verfahren zur Herstellung einer pharmazeutischen Zusammensetzung, umfassend eine

Palladium(II)-Verbindung der in Anspruch 7 angegebenen Forme, und einen pharmakologisch annehmbaren inerten Träger oder ein solches Verdünnungsmittel; in welchem Verfahren eine Palladium(II)-Verbindung der in Anspruch 7 angegebenen Formel und ein pharmakologisch annehmbarer inerter Träger oder ein solches Verdünnungsmittel miteinander vermischt werden.

9. Verwendung einer Palladium(II)-Verbindung der in Anspruch 7 angegebenen Formel in der Herstellung eines Medikamentes für die Behandlung von Tumoren.

**Revendications**

1. Composé de palladium (II) de formule:

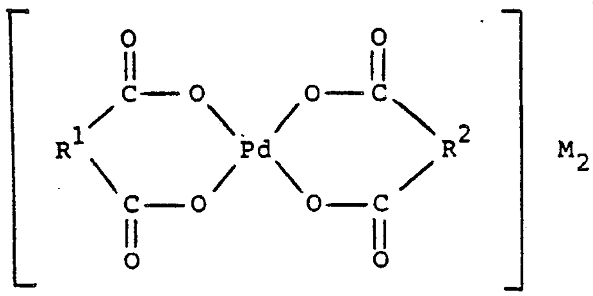

dans laquelle $R^1$ et $R^2$ sont identiques ou différentes et représentent:

(a) un groupe méthylène ou éthylene, à condition que $R^1$ et $R^2$ ne puissent pas représenter tous les deux en même temps le groupe méthylène;

(b) un groupe cycloalkylène de formule:

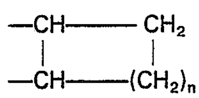

dans laquelle n est un nombre entier ayant une valeur de 2—3;

(c) un groupe alkylidène de 2 à 4 atomes de carbone;

(d) un groupe cycloalkylidène contenant 4 à 6 atomes de carbone dans le cycle;

(e) un groupe o-phénylène de formule:

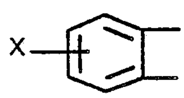

(f) un groupe aralkylidène de formule:

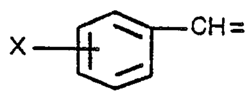

dans lesquelles X est choisi parmi un hydrogène, un halogène et un groupe alkyle en $C_{1-6}$;

(g) un groupe méthylidène substitué de formule $=CR^3R^4$ dans laquelle $R^3$ et $R^4$ sont choisis parmi un hydrogène, un halogène et un hydroxy, à condition que $R^3$ et $R^4$ ne soient pas simultanément un hydrogène ou un hydroxy et que, lorsque $R^3$ ou $R^4$ est un halogène, $R^4$ ou $R^3$ ne soit pas alors un hydroxy; ou

(h) un groupe céto; et

M est un métal alcalin ou $M_2$ est un métal alcalino-terreux.

2. Composé selon la revendication 1, dans lequel le composé de palladium (II) a une structure de formule:

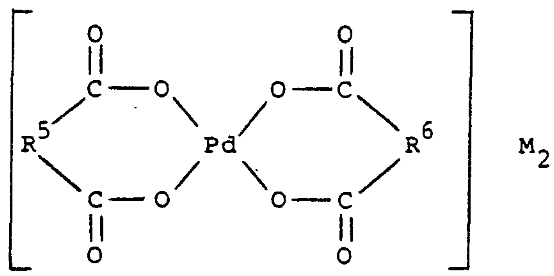

dans laquelle $R^5$ et $R^6$ sont des groupements identiques choisis parmi:
un groupe alkylidène de 2 à 4 atomes de carbone;
un groupe cycloalkylidène contenant de 4 à 6 atomes de carbone dans le cycle;
un groupe o-phénylène de formule:

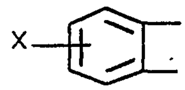

dans laquelle X est choisi parmi un hydrogène, un halogène et un groupe alkyle en $C_{1-6}$;
un groupe hydroxyméthylidène;
un groupe céto; et
M est un métal alcalin.

3. Composé selon la revendication 2, dans lequel $R^5$ et $R^6$ sont choisis parmi les groupes éthylidène, o-phénylène, hydroxyméthylidène et céto.

4. Composé de palladium (II) utilisable dans le traitement des cellules de tumeurs malignes, ledit composé ayant pour formule:

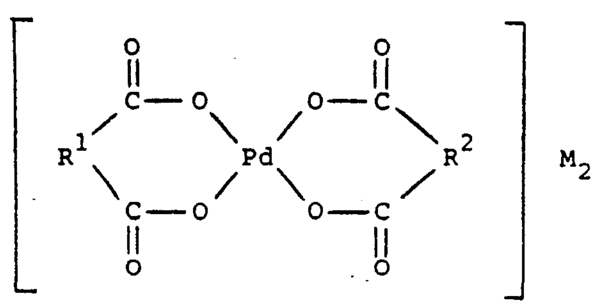

dans laquelle $R^1$ et $R^2$ sont identiques ou différents et représentent:
(a) un groupe méthylène ou éthylene;
(b) un groupe cycloalkylène de formule:

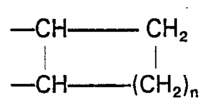

dans laquelle n est un nombre entier ayant une valeur de 2—3;
(c) un groupe alkylidène de 2 à 4 atomes de carbone;
(d) un groupe cycloalkylidène contenant 4 à 6 atomes de carbone dans le cycle;
(e) un groupe o-phénylène de formule:

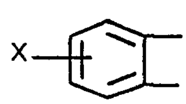

(f) un groupe aralkylidène de formule:

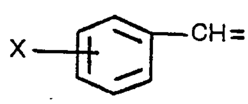

dans lesquelles X est choisi parmi un hydrogène, un halogène et un groupe alkyle en $C_{1-6}$;

(g) un groupe méthylidène substitué de formule $=CR^3R^4$ dans laquelle $R^3$ et $R^4$ sont choisis parmi un ensemble constitué par un hydrogène, un halogène et un hydroxy, à condition que $R^3$ et $R^4$ ne soient pas simultanément un hydrogène ou un hydroxy et que, lorsque $R^3$ ou $R^4$ est un halogène, $R^4$ ou $R^3$ ne soit pas alors un hydroxy; ou

(h) un groupe céto; et

M est un métal alcalin ou $M_2$ est un métal alcalino-terreux.

5. Composé selon la revendication 4, dans lequel $R^1$ et $R^2$ sont identiques.

6. Composition pharmaceutique comprenant un composé de palladium (II) de formule

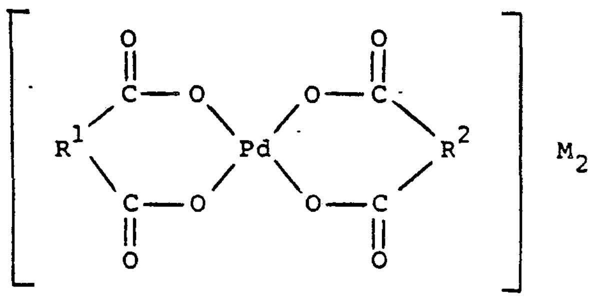

dans laquelle $M_2$, $R^1$ et $R^2$ sont tels que définis dans la revendication 4 ou dans la revendication 5, et un véhicule ou un diluant inerte pharmacologiquement acceptable.

7. Procédé de préparation d'un composé de palladium (II) de formule:

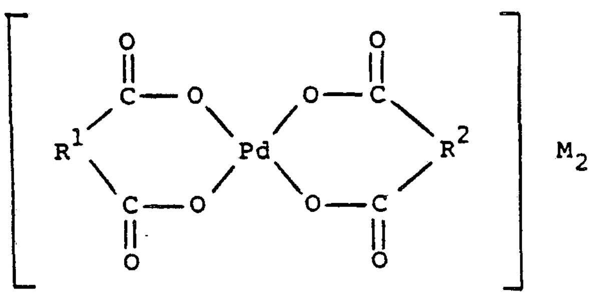

dans laquelle $R^1$ et $R^2$ sont identiques ou différents et représentent:

(a) un groupe méthylène ou éthylène, à condition que $R^1$ et $R^2$ ne puissant pas représenter tous les deux en même temps un groups méthylène;

(b) un groupe cycloalkylène de formule:

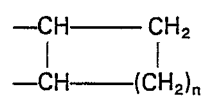

dans laquelle n est un nombre entier ayant une valeur de 2—3;

(c) un groupe alkylidène de 2 à 4 atomes de carbone;

(d) un groupe cycloalkylidène contenant 4 à 6 atomes de carbone dans le cycle;

(e) un groupe o-phénylène de formule:

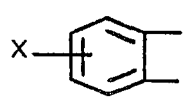

(f) un groupe aralkylidène de formule:

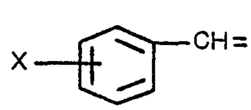

dans lesquelles X est choisi parmi un hydrogène, un halogène et un groupe alkyle en $C_{1-6}$;

(g) un groupe méthylidène substitué de formule $=CR^3R^4$ dans laquelle $R^3$ et $R^4$ sont choisis parmi un

hydrogène, un halogène et un hydroxy, à condition que $R^3$ et $R^4$ ne soient pas simultanément un hydrogène ou un hydroxy et que, lorsque $R^3$ ou $R^4$ est un halogène, $R^4$ ou $R^3$ ne soit pas alors un hydroxy; ou

(h) un groupe céto; et

M est un métal alcalin ou $M_2$ est un métal alcalino-terreux;

ce procédé comprenant le traitement du matériau de départ dicarboxylate $R^1(COOM)_2$, ou $R^1(COOM)_2$ et $R^2(COOM)_2$, où $R^1$ et $R^2$ sont différents, avec un sel de palladium.

8. Procédé de préparation d'une composition pharmaceutique comprenant un composé de palladium (II) de formule indiquée dans la revendication 7, et un véhicule ou un diluant inerte pharmacologiquement acceptable, procédé dans lequel un composé de palladium (II) de formule indiquée dans la revendication 7 et un véhicule ou diluant inerte pharmacologiquement acceptable sont mélangés l'un avec l'autre.

9. Utilisation d'un composé de palladium (II) de formule indiquée dans la revendication 7 dans la préparation d'un médicament pour le traitement des tumeurs.